# EUROPEAN PATENT SPECIFICATION

(11) **EP 2 573 864 B1**
(45) Date of publication and mention of the grant of the patent: **16.10.2019**
(21) Application number: 11857978.8
(22) Date of filing: 27.10.2011
(51) Int. Cl.: H01Q 15/00, H01Q 17/00, H05K 9/00, A61N 1/16

(54) **MAN-MADE MICROSTRUCTURE AND ARTIFICIAL ELECTROMAGNETIC MATERIAL**
KÜNSTLICHE MIKROSTRUKTUR UND KÜNSTLICHES ELEKTROMAGNETISCHES MATERIAL DAMIT
MICROSTRUCTURE ARTIFICIELLE ET MATIÈRE ÉLECTROMAGNÉTIQUE ARTIFICIELLE

(30) Priority: 29.07.2011 CN 201110216571
(43) Date of publication of application: 27.03.2013
(73) Proprietor: Kuang-Chi Innovative Technology Ltd., Shenzhen, Guangdong 518034 (CN); Kuang-Chi Institute of Advanced Technology, Shenzhen, Guangdong 518057 (CN)
(72) Inventor: LIU, Ruopeng, Nanshan District, Shenzhen Guangdong (CN); LUAN, Lin, Nanshan District, Shenzhen Guangdong (CN); KOU, Chaofeng, Nanshan District, Shenzhen Guangdong (CN); YE, Jincai, Nanshan District, Shenzhen Guangdong (CN)
(74) Representative: Mewburn Ellis LLP
(86) International application number: PCT/CN2011/081370
(87) International publication number: WO 2013/016900

(56) References cited:
- WO-A1-00/13040
- WO-A1-2008/083719
- CN-A- 1 627 557
- CN-A- 101 369 680
- CN-A- 101 976 759
- KR-A- 20090 108 994
- US-A1- 2004 140 945
- FILIBERTO BILOTTI ET AL: "Design of Miniaturized Metamaterial Patch Antennas With -Negative Loading", IEEE TRANSACTIONS ON ANTENNAS AND PROPAGATION, IEEE SERVICE CENTER, PISCATAWAY, NJ, US, vol. 56, no. 6, 1 June 2008 (2008-06-01), pages 1640-1647, XP011216005, ISSN: 0018-926X
- KISTENMACHER ET AL: "An Innovative Unit Cell Guide to Multimodal Frequency-Selective Surfaces", ANTENNAS AND PROPAGATION SOCIETY INTERNATIONAL SYMPOSIUM 2006, IEEE ALBUQUERQUE, NM, USA 09-14 JULY 2006, PISCATAWAY, NJ, USA,IEEE, PISCATAWAY, NJ, USA, 1 January 2006 (2006-01-01), pages 4175-4178, XP031017879, DOI: 10.1109/APS.2006.1711549 ISBN: 978-1-4244-0123-9
- SUI, QIANG ET AL.: 'Experiments On Negative Permittivity and Microwave Medium of Negative Magnetic Permeability' SCIENCE IN CHINA (SERIES G) vol. 33, no. 5, October 2003, pages 416 - 427, XP008171319
- CHENG, QAING ET AL.: 'Application of Planar Left-Handed Circuits on the Design of Microwave Components and Antennas' CHINESE JOURNAL OF ELECTRONICS vol. 35, no. 6A, June 2007, pages 118 - 126, XP008171320
- BILOTTI F ET AL: "Design of Spiral and Multiple Split-Ring Resonators for the Realization of Miniaturized Metamaterial Samples", IEEE TRANSACTIONS ON ANTENNAS AND PROPAGATION, IEEE SERVICE CENTER, PISCATAWAY, NJ, US, vol. 55, no. 8, 1 August 2007 (2007-08-01) , pages 2258-2267, XP011189629, ISSN: 0018-926X, DOI: 10.1109/TAP.2007.901950
- VIDYALAKSHMI M R ET AL: "A CAD model of triangular Split Ring Resonator based on equivalent circuit approach", APPLIED ELECTROMAGNETICS CONFERENCE (AEMC), 2009, IEEE, PISCATAWAY, NJ, USA, 14 December 2009 (2009-12-14), pages 1-4, XP031898004, DOI: 10.1109/AEMC.2009.5430633 ISBN: 978-1-4244-4818-0
- CUMALI SABAH: "TUNABLE METAMATERIAL DESIGN COMPOSED OF TRIANGULAR SPLIT RING RESONATOR AND WIRE STRIP FOR S- AND C- MICROWAVE BANDS", PROGRESS IN ELECTROMAGNETICS RESEARCH B, vol. 22, 1 January 2010 (2010-01-01), pages 341-357, XP055400676, DOI: 10.2528/PIERB10051705

## Description

The present invention relates to electromagnetic field, and more particularly to an artificial microstructure and an artificial electromagnetic material using the same.

### BACKGROUND OF THE INVENTION

In prior art, CN 101976759A discloses an equivalent LHM (Left Handed Material) patch antenna of split ring resonators. The patch antenna comprises two dielectric substrates, a metal grounding plate, two square metal radiation sheets and a metal feeder, wherein the metal grounding plate is fixed on the back of the second dielectric substrate, the square metal radiation sheets are fixed on the front of the first dielectric substrate, the metal feeder is connected with the metal grounding plate and the metal radiation sheets, a four-layered split ring resonator is carved in the square metal radiation sheet on the front of the first dielectric substrate, and another four-layered split ring resonator is carved in the square metal radiation sheet on the front of the second dielectric substrate. In this document, an LHM combination is added to the patch dielectric substrates to form a patch antenna, thus the localization degree of electromagnetic energy is remarkably improved so as to have higher gain, present lower echo loss, favourably improve the antenna performance to ensure that the patch antenna can be better applied to a plurality of fields, such as mobile communication, satellite communication, aerospace, and the like.

KR 20090108994A discloses a low phase noise VCO using microstrip square open loop multiple split ring resonator is provided to improve phase noise characteristic without the reduction of frequency adjusting range. The microstrip square open loop multiple split ring resonator is designed to combine the SRR with a high Q value microstrip rectangular open loop resonator. The electric field and magnetic field characteristics of the two resonators are combined complementarily. The SRR is rectangular. The rectangular shape is in favour of the easy combining between the micro rectangular open loop resonator and the SRR. Multiple SRR is formed to increase the coupling coefficient of the SRR.

The article entitled "Design of Miniaturized Metamaterial Patch Antennas With-Negative Loading", from IEEE TRANSACTIONS ON ANTENNAS AND PROPAGATION, IEEEE SERVICE CENTER, PISCATAWAY, NJ, US, vol. 56, no. 6, 1 June 2008 (2008-06-01), pages 1640-1647, XP011216005, ISSN: 0018-926X discloses recent theoretical studies have shown that circular patch antennas loaded by an inhomogeneous substrate partially filled with MNG metamaterial may in principle support a resonant radiating mode, even if the total size of the radiator is significantly smaller than the wavelength of operation.

US 2004/140945A1 discloses configuring a high impedance frequency selective surface (HZ-FSS) structure for the appropriate values of surface impedance (surface resistance and surface reactance) in order to synthesize a high frequency artificial ferrite metamaterial with almost any desired value of real and imaginary permeability. Materials with these properties have not previously been physically realizable at frequencies above 1 GHz.

An article entitled "An Innovative Unit Cell Guide to Multimodal Frequency-Selective Surfaces", from ANTENNAS AMD PROPAGATION SOCIETY INTERNATIONAL SYMPOSIUM 2006, IEEE ALBUQUERQUE, NM, USA 09-14 JULY 2006, PISCATAWAY, NJ, USA, 1, January 2006 (2006-01-01), pages 4175-4178, XP031017879, ISSN: 978-1-4244-0123-9 discloses over the past several years, there has been considerable interest in frequency-selective surfaces with applications ranging from RFID tags to multimode array antennas. All of these application are predicated on the frequency-selective surface acting as a filter for incident plane-wave radiation, often in the microwave or millimetre regime. Generally, frequency-selective surfaces are based on a two-dimensional periodic lattice decorated with resonant elements-including dielectric or metallic circuit designs. Split ring resonator are becoming important elements of such surfaces. This article discusses experimental and modelling studies on two-dimensional arrays of two elementary resonators: nested split-ring square resonators introduced by Shelby, Smith and Schultz and nested, split-ring triangular resonators introduced from this laboratory.

WO 2008/083719A1 discloses an antenna design in the technical field of Radiofrequency Identification (RFID). The antenna comprises a radiating element that achieves self-resonance without needing any external matching network and can be reduced in size arbitrarily. The radiating element is based on a split ring resonator structure whose overall size can be reduced as much as needed, independently from the resonant frequency required, just by increasing the overall inductance and capacitance between the rings of the structure excited at a feed point. The feeding method can vary to increase the radiation resistance of the antenna or for compensating the capacitive behaviour of an RFID chip. This small antenna is especially suitable for RFID applications because it can be fabricated in a planar substrate, with reduced dimensions typical for conventional antennas used as RFID tags.

The article entitled "Design of Spiral and Multiple Split-Ring Resonators for the Realization of Miniaturized Metamaterial Samples", from IEEE TRANSACTIONS ON ANTENNAS AND PROPAGATION, IEEEE SERVICE CENTER, PISCATAWAY, NJ, US, vol. 55, no. 8, 1 August 2007 (2007-08-01), pages 2258-2267, discloses the design of miniaturized resonant inclusions to be employed in the practical realization of metamaterial samples with anomalous values of the real part of the permeability. The inclusions here considered are the multiple split-ring resonators (MMSRs), that represent a straightforward extension of the commonly used split-ring resonators (SRRs), and the spiral resonators (SRs), that enable a greater miniaturization rate. Some physical insights on the resonance mechanism and on the inherent saturation of the resonant frequency when increasing the number of the rings of the MSRRs and the number of the turns of the SRs are given. New and accurate analytical design formulas, based on a quasi-static model, for both MSRRs and SRs are derived and tested through a proper comparison with the existing formulas and full-wave numerical results. Both MSRRs and SRs are shown to be useful to reduce the electrical dimensions of the resonant inclusions when synthesizing artificial metamaterials.

The article entitled "A CAD model of triangular Split Ring Resonator based on equivalent circuit approach", from APPLIED ELECTROMAGNETICS CONFERENCE (AEMC), 2009, IEEE, PISCATAWAY, NJ, USA, 14 December 2009 (2009-12-14), pages 1-4, discloses a new structure of triangular split ring resonator with edge-coupling is proposed. The structure consists of two equilateral concentric triangles made of metal with dielectric medium filled in the gaps. The advantage of this structure includes the reduction in size of the SRR and the wide range of resonant frequencies possible.

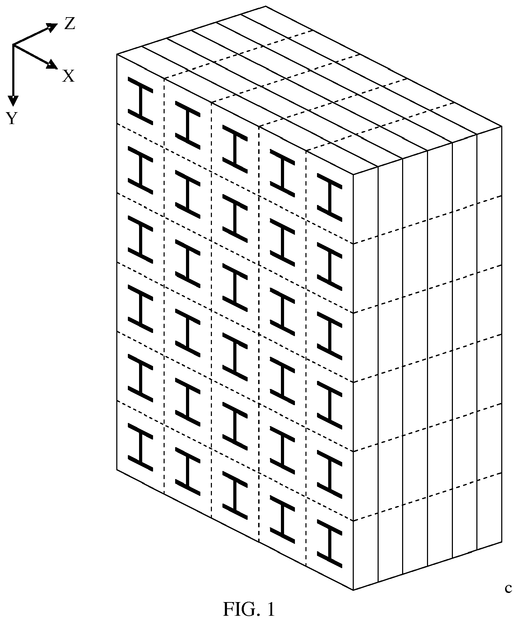

The article entitled "TUNABLE METAMATERIAL DESIGN COMPOSED OF TRIANGULAR SPLIT RING RESONATOR AND WIRE STRIP FOR S- AND C- MICROWAVE BANDS", from PROGRESS IN ELECTROMAGNETICS RESEARCH B, vil. 22, 1 January 2010 (2010-01-01), pages 341-357, discloses three types of new metamaterials, concentric and non-concentric configurations. Figure 1 of this article illustrates the geometry of the unit cells comprised of TSRR and WS for three different structures. The first one is arranged to have a vertex to vertex configuration and the second one is modified to have a base to base arrangement; the third structure is formed from nested TSRR which is considered to have a similar configuration as in the concentric loop-like (circular, square, or etc.) split ring resonator.

With the development of modern science and technology, the effects of electromagnetic radiation on the environment are becoming increasingly serious. At the airport, aircrafts could not take off due to electromagnetic interference and are delayed. In the hospital, a plurality of electronic diagnosis and treatment apparatuses cannot work properly due to interferences of mobile phones. Therefore, controlling electromagnetic pollution and searching for a kind of wave-absorbing material for resisting and weakening electromagnetic radiation have become a major issue in material science.

Wave-absorbing material can effectively absorb incident electromagnetic waves and make incident electromagnetic waves be scattered and attenuated. Wave-absorbing material can convert the incident electromagnetic waves into heat or other forms of energy through various loss mechanisms of the material for the purpose of absorbing wave. Wave-absorbing material includes structural wave-absorbing material and coated wave-absorbing material. The former mainly is wedge-shaped, bubble-shaped, flat-shaped and etc. The latter is compounded by binder and absorbent, the absorbing ability is mostly related to the type of the absorbent. However, each type of the wave-absorbing material has a constant absorbing frequency band which cannot be changed freely.

A new synthetic material, called artificial electromagnetic material (metamaterial), has a feature that it can be designed freely according to different working frequency band, and therefore comes to be a new direction in the study of wave-absorbing material.

The artificial electromagnetic material is new synthetic material which can respond to electromagnetic waves. Referring to FIG. 1, the artificial electromagnetic material includes a substrate and a plurality of artificial microstructures attached to the substrate. Since the artificial microstructures usually have a certain shape such as "I" shape constructed by metal wires, the microstructures can respond to electromagnetic waves, thereby the artificial electromagnetic material reflects different electromagnetic properties from that of the substrate. Designing artificial microstructures with special properties, a broadband wave-absorbing material can be achieved. And if sizes and shape of the artificial microstructures are changed, the absorbing band of the artificial electromagnetic material will be changed.

### SUMMARY OF THE INVENTION

Aiming at the defect that the existing wave-absorbing material has constant absorbing frequency band, a technical problem to be solved in present invention is to provide an artificial microstructure and an artificial electromagnetic material using the same which can absorb wave in a broad frequency band while their absorbing band can be designed.

The present invention provides an artificial electromagnetic material as set out in claim 1. The artificial electromagnetic material includes at least one material sheet that comprises a substrate having a front surface and a back surface, and a plurality of artificial microstructures attached on the front surface of the substrate. A metal foil is attached to the back surface of the substrate. Each of the plurality of artificial microstructures includes at least three split rings, wherein a second split ring is located in a first split ring and a third split ring is located in the second split ring, each split ring is formed by a wire which is made of conductive material, with two terminals of the wire towards each other to form an opening of the corresponding split ring, wherein each split ring is shaped as a triangle, and the openings of adjacent split rings are located at 180 degrees relative to each other around a centre of the respective triangles, wherein the substrate includes a plurality of rectangle substrate units which are the same as each other and are arranged in an array, with each one's front surface attached to one artificial microstructure, and wherein the metal foil is a fishnet structure having meshes that are located on a common junction of four adjacent substrate units.

The metal foil may include a number of the metal foil units, where the number of artificial microstructures is same as the number of metal foil units.

The metal foil units may cover the whole back surface of the corresponding substrate unit except for four corners of the corresponding substrate unit.

The substrate may be made of polyfluortetraethylene or epoxide resin.

There may be several material sheets, which are arranged along a direction perpendicular to the substrate, set in parallel and spaced evenly.

The row spacing and the column spacing of the artificial microstructure array may be either smaller than or equal to one tenth of the wavelength of the incident electromagnetic wave absorbed by the artificial electromagnetic material.

The present invention of artificial electromagnetic material, which can achieve the broadband wave-absorbing, have the following beneficial effects: owing to the front surface of the substrate attached with the artificial microstructures, which includes a plurality of split rings, and the back surface of the substrate attached with the metal foil, electromagnetic waves will reflected by the metal foil to re-enter the substrate after passing through the artificial microstructure. The electromagnetic waves are absorbed repeatedly. Therefore, the artificial electromagnetic material can achieve broadband wave-absorbing in high efficiency.

Other advantages and novel features of the present disclosure will become more apparent from the following detailed description of preferred embodiment when taken in conjunction with the accompanying drawings.

### BRIEF DESCRIPTION OF THE DRAWINGS

The accompanying drawings facilitate an understanding of the various embodiments of this invention. In such drawings:
FIG. 1 is a schematic diagram of an embodiment of a conventional artificial electromagnetic material.
FIG. 2 is a front view of a first example of an artificial electromagnetic material.
FIG. 3 is a back view of the artificial electromagnetic material of FIG. 2.
FIG. 4 is a front view of a material unit of the artificial electromagnetic material of FIG. 2.
FIG. 5 is a back view of the material unit of FIG. 4.
FIG. 6 is a schematic diagram of a second example of a conventional artificial microstructure.
FIG. 7 is a schematic diagram of an artificial microstructure according to a third embodiment.
FIG. 8 is a schematic diagram of an artificial microstructure according to a forth embodiment.
FIG. 9 is a schematic diagram of an artificial microstructure according to a fifth embodiment according to the invention.
FIG. 10 is a schematic diagram of an artificial microstructure according to a sixth embodiment according to the invention.

### DETAILED DESCRIPTION OF ILLUSTRATED EMBODIMENTS

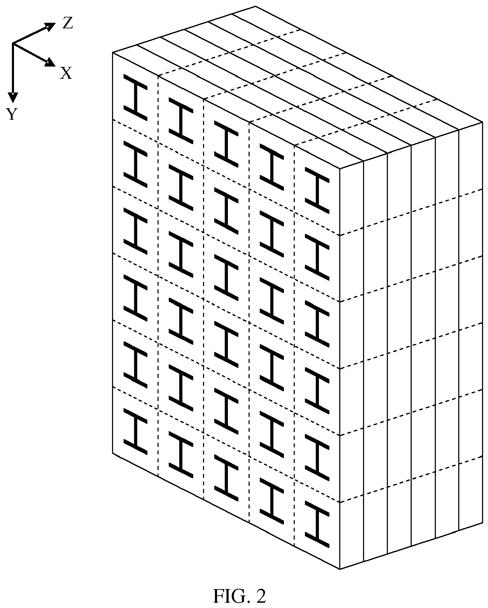

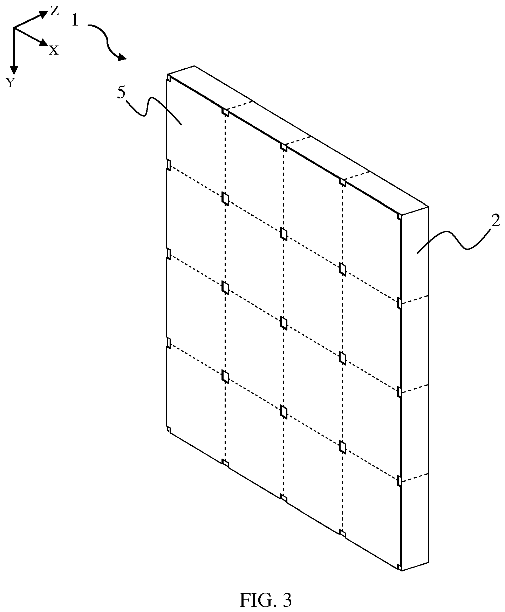

Referring to FIGs. 2 and 3, the present invention relates to an artificial electromagnetic material for broadband wave-absorbing. The artificial electromagnetic material includes at least one material sheet 1. Each material sheet 1 includes a substrate 2 and a plurality of artificial microstructures 3 attached to a surface of the substrate 1. The plurality of artificial microstructures 3 are periodically arranged on the surface of the substrate 2, such as arranged in rectangular array. In the rectangular arrays, X direction is defined as the direction of rows, Y direction perpendicular to X direction is defined as the direction of columns. In the rectangular array, the row spacings are the same and the column spacings are the same. Even the row spacing and the column spacing are equal. Preferably, the row spacing and the column spacing are not greater than one fifth of the wavelength of the absorbed incident electromagnetic wave. Preferably, the row spacing and the column spacing are equal to one tenth of the wavelength of the absorbed incident electromagnetic wave. If there are several material sheets 1, the material sheets 1 are arranged along Z direction perpendicular to the substrate 2 in turn. The material sheets 1 are set in parallel. Preferably, the material sheets 1 are parallel and spaced evenly.

In the artificial electromagnetic material, the artificial microstructure 3 is attached to a front surface of the substrate 2. Metal foil 5 is attached to a back surface of the substrate 2. The thickness of the metal foil 5 is substantially in a range of 0.018-0.035mm. The metal foil 5 is usually made of copper or silver. The metal foil 5 can reflect the electromagnetic wave transmitted into the substrate 2 to make the electromagnetic wave re-enter the substrate 2. The substrate 2 absorbs the reentered electromagnetic wave. The times of the electromagnetic wave entering the substrate 2 increases, which results in the electromagnetic wave are repeatedly absorbed. Therefore, the ability of absorbing waves is improved.

The metal foil 5 can be as a whole slice of foil film which is attached to the back surface of the substrate 2. Preferably, the metal foil 5 is fish net shaped, that is, a plurality of meshes are evenly defined in the whole slice of foil film. The plurality of meshes are located on the common junction of the four adjacent substrate units.

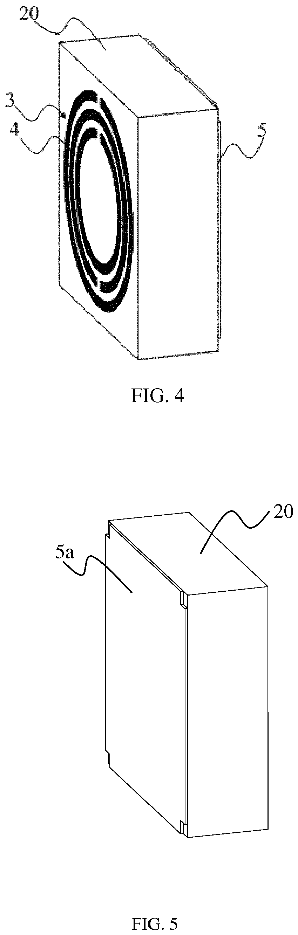

Referring to FIGs. 4 and 5, the artificial microstructure 3 is arranged in an array, so the substrate 2 can be virtually divided into a
number of substrate units 20 arranged in the same array. The length of each substrate unit 20 is equal to the above-mentioned row spacing. The width of each substrate unit 20 is equal to the above-mentioned column spacing. The thickness of each substrate unit 20 is equal to the thickness of the substrate 2. Surface of each substrate unit 20 just corresponds to one artificial microstructure 3.

Metal foil 5 is virtually divided into a plurality of metal foil units 5a according to the same division rule of the metal unit. The metal foil units 5a are arranged in multiple arrays, in which X direction is defined as the direction of the line, and Y direction is defined as the direction of the row. Referring to FIG.5, each metal foil unit 5a covers the whole back surface of the corresponding substrate unit 20 except for four corners of the back surface of the corresponding substrate unit 20. One mesh is formed by one corner and the corners of the adjacent three metal foil units 5a together (see FIG.3). Preferably, each artificial microstructure 3 is the same and each metal foil unit 5a is the same. That achieves the uniform absorption effect on any positions of the substrate 2.

The metal foil 5 with the meshes is applied to make the electromagnetic waves passing through the meshes produce electromagnetic effects, to further cause the accumulation of quantity of electron. Therefore, capacitive effect between the metal foil 5 and former structure is enhanced. The metal foil 5 has a great reflection effect for the electromagnetic waves. Therefore, the transmission distance of the electromagnetic waves in the substrate 2 increases and the wave-absorbing effect also increases.

The advantages of the present invention are that the shape of the artificial microstructure 3 is a new geometry pattern. The artificial microstructure 3 includes at least three split rings 4. The at least three split rings 4 surround and embed in turn. That is, a second split ring is located in a first split ring. A third split ring is located in the second split ring, and the rest of the split rings can be located in the same manner. Each split ring is formed by a wire which is made of conductive material, with two terminals of the wire towards each other to form an opening of the corresponding split ring. The conductive material can be metal material, such as silver, copper, copper alloy, and so on. The conductive material also can be non-metal material such as conductive plastic, and so on. The at least three split rings 4 which are surrounded and embedded in turn can realize the function of broadband wave-absorbing.

FIG. 4 is a schematic diagram of the first example of the conventional artificial microstructure. The artificial microstructure includes three split rings 4. Each split ring 4 is a circular split ring. That is, the split ring, formed by a wire with the two terminals of the wire are connected together, is circular ring. Preferably, each circular split ring is arranged concentrically. That is, every circular split ring 4 has the same centre of a circle, and the centre angle between the openings of the two adjacent circular split rings 4 related to the centre of the circle is equal to 180 degrees.

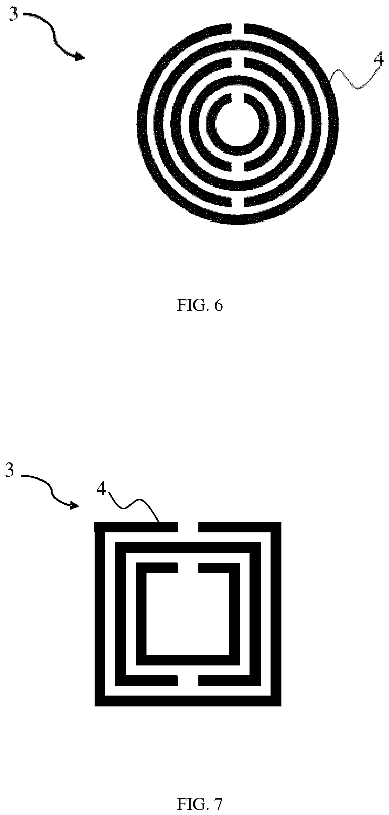

FIG. 6 is a schematic diagram of a second example of a conventional artificial microstructure, and similar to the conventional artificial microstructure of the first embodiment. A difference between the second embodiment and the first embodiment of the artificial microstructure is the number of the split rings 4. In the second embodiment, the number of the split rings 4 is five.

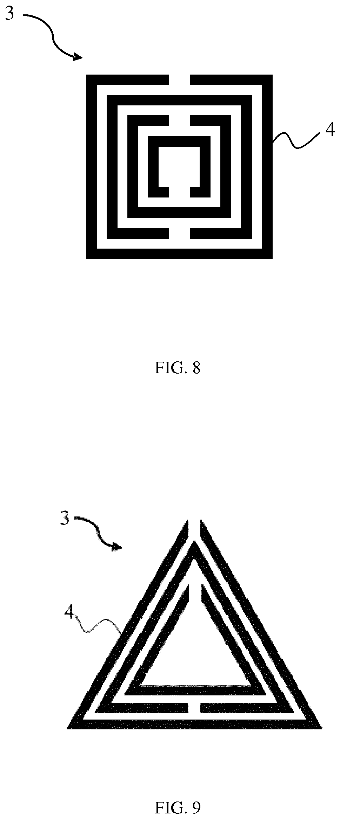

Similarly, FIG.7 is a schematic diagram of a third embodiment of the conventional artificial microstructure. FIG.8 is a schematic diagram of a fourth embodiment of the conventional artificial microstructure. A difference among the third, fourth embodiments, and the first embodiment is the shape of the split ring 4 of the artificial microstructure 3. In the third and fourth embodiments, the split ring 4 is a rectangle. Sides of the split rings 4 are parallel correspondingly. In the third embodiment, the number of the split rings 4 is three. In the fourth embodiment, the number of the split rings 4 is four. Preferably, the openings of the two adjacent split rings 4 are defined away from each other. That is, the opening of one of the two adjacent split rings 4 is defined in a rectangle side. The opening of the other of the adjacent two split rings 4 is defined in a rectangle side parallel to the rectangle side of the former split ring 4.

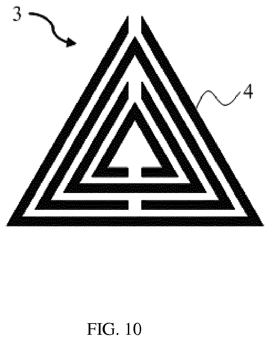

Similarly, FIG.9 is a schematic diagram of an artificial microstructure of a fifth embodiment according to the invention. FIG. 10 is a schematic diagram of an artificial microstructure of a sixth embodiment of the invention. A difference among these embodiments of the artificial microstructure of the present invention, and the first embodiment of the conventional artificial microstructure is the shape of the split rings 4. In the fifth and sixth embodiment according to the present invention, the split ring 4 is a triangle. In the fifth embodiment according to the present invention, the number of the triangle split rings 4 is three. In the sixth embodiment according to the present invention, the number of the triangle split rings 4 is four. Preferably, the openings of the two adjacent split rings 4 face to each other. That is, the opening of one of the two adjacent split rings 4 is defined in a vertex angle of the split ring 4. The opening of the other of the two adjacent split rings 4 is defined in a bottom opposite to the vertex angle of the split ring 4.

In general and not according to the invention the shapes of the at least three split rings 4 of the artificial microstructure 3 are not limited to only satisfy the above described three shapes. The shapes of the at least three split rings 4 of the artificial microstructure 3 can be other arbitrary shapes. The at least three split rings 4 of the artificial microstructure 3 are not necessary the same shapes or geometrically similar. The at least three split rings 4 of the artificial microstructure 3 can be a mixture of the above described all kinds of the split rings 4, or a mixture of other split rings of irregular shape.

Artificial electromagnetic material of the present invention has the effect of broadband wave-absorbing. For example, in the first embodiment, the substrate 2 is made of FR-4 epoxy resin material. The size of the substrate unit 20 is 6mm × 6mm × 0.4mm, wherein 0.4mm is the thickness of the substrate 2. The outer diameter of the three split rings 4 are respectively 5.9mm, 5.6mm, and 5.3mm. The wire of the split rings 4 is made of copper. The width of the wire is substantially 0.2mm. The thickness of the wire is substantially 0.018mm. The metal foil unit 5 is copper foil. The thickness of the copper foil is substantially 0.018mm. Each of the uncoated Areas located in the four corners is substantially 0.1mm × 0.1mm.

After simulation on above-mentioned artificial electromagnetic material formed by the substrate units, the artificial electromagnetic material can absorb at least 80% of the electromagnetic wave of which
the frequency range is from 15.05GHz to 15.42GHz. Therefore, the above-mentioned embodiment of the artificial electromagnetic material has a great effect for absorbing electromagnetic wave of which the frequency range is from 15.05GHz to 15.42GHz., to achieve broadband wave-absorbing.

While the invention has been described in connection with what are presently considered to be the most practical and preferred embodiments, it is to be understood that the invention is not to be limited to the disclosed embodiments and defined by the following claims.

## Claims

1. An artificial electromagnetic material comprising:
at least one material sheet (1) comprising:
a substrate (2) comprising a front surface and a back surface; and
a plurality of artificial microstructures (3) attached on the front surface of the substrate (2);
wherein a metal foil (5) is attached to the back surface of the substrate (2),
wherein each artificial microstructure comprises at least three split rings (4), wherein a second split ring is located in a first split ring and a third split ring is located in the second split ring, each split ring (4) being formed by a wire which is made of conductive material, with two terminals of the wire directed towards each other to form an opening of the corresponding split ring,
wherein each split ring (4) is shaped as a triangle, and the openings of adjacent split rings (4) are located at 180 degrees relative to each other around a centre of the respective triangles,
wherein the substrate (2) comprises a plurality of rectangular substrate units (20) which are the same as each other and are arranged in an array, with each one's front surface attached to one artificial microstructure (3), and
wherein the metal foil (5) has a fishnet structure having meshes that are located on a common junction of four adjacent substrate units (20).

2. The artificial electromagnetic material of claim 1, wherein the metal foil (5) comprises a number of the metal foil units (5a), wherein the number of artificial microstructures (3) is same as the number of metal foil units (5a), and wherein each of the plurality of rectangular substrate units (20) has a back surface attached to one metal foil unit (5a).

3. The artificial electromagnetic material of claim 2, wherein the metal foil unit (5a) covers the whole back surface of the corresponding substrate unit (20) except for four corners of the corresponding substrate unit (20).

4. The artificial electromagnetic material of claim 1, wherein the substrate (2) is made of polyfluortetraethylene or epoxide resin.

5. The artificial electromagnetic material of claim 1, wherein there are several material sheets (1), which are arranged along a direction perpendicular to the substrate (2), set in parallel and spaced evenly.

6. The artificial electromagnetic material of claim 1, wherein a row spacing and a column spacing of the artificial microstructure array (3) are either smaller than or equal to one tenth of the wavelength of the incident electromagnetic wave absorbed by the artificial electromagnetic material.

## Patentansprüche

1. Künstliches elektromagnetisches Material, umfassend:
mindestens eine Materialplatte (1), die Folgendes umfasst:
ein Substrat (2), das eine Vorderseitenfläche und eine Rückseitenfläche umfasst; und
eine Vielzahl von künstlichen Mikrostrukturen (3), die auf der Vorderseitenfläche des Substrats (2) befestigt sind;
wobei eine Metallfolie (5) an der Rückseitenfläche des Substrats (2) befestigt ist,
wobei jede künstliche Mikrostruktur mindestens drei Spaltringe (4) umfasst, wobei ein zweiter Spaltring in einem ersten Spaltring angeordnet ist, und ein dritter Spaltring in dem zweiten Spaltring angeordnet ist, wobei jeder Spaltring (4) durch einen aus leitfähigem Material hergestellten Draht ausgebildet ist, wobei zwei Endstücke des Drahts in Richtung zueinander ausgerichtet sind, um eine Öffnung des entsprechenden Spaltrings zu bilden,
wobei jeder Spaltring (4) als ein Dreieck geformt ist, und die Öffnungen von angrenzend angeordneten Spaltringen (4) bei 180 Grad relativ zueinander um einen Mittelpunkt der jeweiligen Dreiecke angeordnet sind,
wobei das Substrat (2) eine Vielzahl von rechteckigen Substrateinheiten (20) umfasst, die untereinander gleich sind und in einer Anordnung angeordnet sind, wobei deren Vorderseitenfläche jeweils an einer künstlichen Mikrostruktur (3) befestigt ist, und
wobei die Metallfolie (5) eine Fischnetzstruktur mit Maschen aufweist, die auf einer gemeinsamen Verbindungsstelle von vier angrenzend angeordneten Substrateinheiten (20) angeordnet sind.

2. Künstliches elektromagnetisches Material nach Anspruch 1, wobei die Metallfolie (5) eine Anzahl von Metallfolieneinheiten (5a) umfasst, wobei die Anzahl von künstlichen Mikrostrukturen (3) dieselbe wie die Anzahl von Metallfolieneinheiten (5a) ist, und wobei jede aus der Vielzahl von rechteckigen Substrateinheiten (20) eine Rückseitenfläche aufweist, die an einer Metallfolieneinheit (5a) befestigt ist.

3. Künstliches elektromagnetisches Material nach Anspruch 2, wobei die Metallfolieneinheit (5a) mit Ausnahme von vier Ecken der entsprechenden Substrateinheit (20) die gesamte Rückseitenfläche der entsprechenden Substrateinheit (20) abdeckt.

4. Künstliches elektromagnetisches Material nach Anspruch 1, wobei das Substrat (2) aus Polyfluortetraethylen oder Epoxidharz hergestellt ist.

5. Künstliches elektromagnetisches Material nach Anspruch 1, wobei mehrere Materialplatten (1) vorhanden sind, die entlang einer zum Substrat (2) senkrechten Richtung angeordnet, parallel ausgerichtet und gleichmäßig voneinander beabstandet sind.

6. Künstliches elektromagnetisches Material nach Anspruch 1, wobei ein Zeilenabstand und ein Spaltenabstand der künstlichen Mikrostrukturanordnung (3) entweder kleiner als ein oder gleich einem Zehntel der Wellenlänge der einfallenden elektromagnetischen Welle sind, die von dem künstlichen elektromagnetischen Material absorbiert wird.

## Revendications

1. Matériau électromagnétique artificiel comprenant :
au moins une feuille de matériau (1) comprenant :
un substrat (2) comprenant une surface avant et une surface arrière ; et
une pluralité de microstructures artificielles (3) fixées sur la surface avant du substrat (2) ;
dans lequel une feuille métallique (5) est fixée à la surface arrière du substrat (2),
dans lequel chaque microstructure artificielle comprend au moins trois anneaux fondus (4), dans lequel un deuxième anneau fendu est situé dans un premier anneau fendu et un troisième anneau fendu est situé dans le deuxième anneau fendu, chaque anneau fendu (4) étant formé par un fil qui est réalisé en un matériau conducteur, avec deux bornes du fil dirigées l'une vers l'autre pour former une ouverture de l'anneau fendu correspondant,
dans lequel chaque anneau fendu (4) est doté de la forme d'un triangle, et les ouvertures d'anneaux fendus adjacents (4) sont situées à 180 degrés les unes par rapport aux autres autour d'un centre des triangles respectifs,
dans lequel le substrat (2) comprend une pluralité d'unités de substrat rectangulaires (20) qui sont les mêmes les unes aux autres et sont agencées en un réseau, la surface avant de chacun étant fixée à une microstructure artificielle (3), et
dans lequel la feuille métallique (5) a une structure en filet ayant des mailles qui sont situées sur une jonction commune de quatre unités de substrat adjacentes (20).

2. Matériau électromagnétique artificiel selon la revendication 1, dans lequel la feuille métallique (5) comprend un certain nombre d'unités de feuille métallique (5a), dans lequel le nombre de microstructures artificielles (3) est le même que le nombre d'unités de feuille métallique (5a), et dans lequel chacune de la pluralité d'unités de substrat rectangulaires (20) a une surface arrière fixée à une unité de feuille métallique (5a).

3. Matériau électromagnétique artificiel selon la revendication 2, dans lequel l'unité de feuille métallique (5a) recouvre la surface arrière totale de l'unité de substrat correspondante (20), à l'exception de quatre coins de l'unité de substrat correspondante (20).

4. Matériau électromagnétique artificiel selon la revendication 1, dans lequel le substrat (2) est constitué de polyfluorotétraéthylène ou d'une résine époxyde.

5. Matériau électromagnétique artificiel selon la revendication 1, dans lequel il existe plusieurs feuilles de matériau (1), qui sont disposées dans une direction perpendiculaire au substrat (2), placées parallèlement et espacées régulièrement.

6. Matériau électromagnétique artificiel selon la revendication 1, dans lequel un espacement de rangées et un espacement de colonnes du réseau de microstructures artificielles (3) sont inférieurs ou égaux à un dixième de la longueur d'onde de l'onde électromagnétique incidente absorbée par le matériau électromagnétique artificiel.
